# EUROPEAN PATENT SPECIFICATION

(11) **EP 3 505 205 B1**
(45) Date of publication and mention of the grant of the patent: **30.11.2022**
(21) Application number: 17843596.2
(22) Date of filing: 22.08.2017
(51) Int. Cl.: A61M 5/303, A61M 5/20, A61M 5/30, A61M 5/31

(54) **ADMINISTRATION APPARATUS**
VERABREICHUNGSVORRICHTUNG
APPAREIL D'ADMINISTRATION

(30) Priority: 23.08.2016 JP 2016162872
(43) Date of publication of application: 03.07.2019
(73) Proprietor: Daicel Corporation, Osaka-shi, Osaka 530-0011 (JP)
(72) Inventor: MASUMOTO, Takaya, Tatsuno-shi, Hyogo 671-1681 (JP)
(74) Representative: Jones, Nicholas Andrew
(86) International application number: PCT/JP2017/029983
(87) International publication number: WO 2018/038118

(56) References cited:
- EP-A1- 2 959 931
- EP-A2- 0 963 211
- JP-A- 2004 500 933
- JP-A- 2011 067 265
- JP-A- 2013 053 654
- JP-A- 2016 151 318
- JP-A- 2016 151 318
- JP-A- 2017 519 570

## Description

### [Technical Field]

The present invention relates to an administration apparatus that administers a substance to be administered to an administration target area.

### [Background Art]

An example of an administration apparatus that administers a substance to be administered includes an injector. There are cases where gunpowder is used as a pressurization source in a needleless injector that performs injection without the intervention of an injection needle (see, e.g., PTL 1). In the needleless injector described in PTL 1, a detonator and explosive charge are provided, the detonator is pierced with a hammer, and the detonator ignites, whereby thermal energy generated by the ignition is transmitted to the explosive charge. Subsequently, the explosive charge is combusted, whereby an injection solution is pressurized. As the explosive charge described above, nitrocellulose-based single gunpowder is used.

Combustion energy of gunpowder is used as a power source for pressurization also in a field different from the field of the injector. For example, PTL 2 discloses a technique related to an actuator for driving a control member via a membrane by using the combustion energy of the gunpowder to interrupt the flow of a medium in a channel. In the technique, the elastically deformable membrane sandwiched between the control member and a housing receives the combustion pressure of the gunpowder and is thereby deformed, a cylinder portion attached to the membrane is displaced, and the control member is thereby driven.

### [Citation List]

### [Patent Literature]

[PTL 1] Japanese Translation of PCT Application No. 2004-532049
[PTL 2] U.S. Patent Specification No. 6397595

JP 2016 151318 A discloses a pyro-type actuator mechanism, injection unit and igniter assembly. EP 0 963 211 A2 discloses a hypodermic injection system. EP 2 959 931 A1 discloses a needle-free injection device.

### [Summary of Invention]

### [Technical Problem]

In the case where the combustion energy of the gunpowder is used as the power source for pressurization, it is necessary to consider influences of combustion products generated by the combustion such as combustion gas and a combustion residue. For example, in the case where the gunpowder is used as the pressurization source for an object in the administration apparatus that administers the substance to be administered, it is not preferable that the combustion product is mixed into the substance to be administered serving as a pressurization target.

On the other hand, as in the conventional art, in the case where a space in which the combustion of the gunpowder is performed is separated from a space where the substance to be administered that is pressurized is disposed by the elastically deformable membrane, and the combustion energy of the gunpowder is transmitted to the substance to be administered via the deformation of the membrane, the membrane is elastically deformed sharply at the time of the combustion. In addition, for the pressurization, the membrane needs to secure a desired deformation amount in the deformation. However, in the conventional art, the membrane needs to be elastically deformed significantly toward the control member by the combustion of the gunpowder, and it is feared that the membrane may be damaged or torn in some situations. When the membrane is damaged, it becomes difficult to seal the combustion product in the space on the side where the combustion is performed. In addition, the elastically deformable membrane according to the conventional art is deformed so as to stick to a specific surface in an internal space. However, depending on the deformation of the membrane, the membrane can not necessarily press the control member adequately, and adequate transmission of the combustion energy to the control member may be hindered.

To cope with this, in view of the above problem, an object of the present invention is to prevent, in an administration apparatus that administers a substance to be administered using gunpowder combustion, a combustion product generated by the gunpowder combustion from acting on the substance to be administered and suitably transmit energy for administration to the substance to be administered.

### [Solution to Problem]

In order to solve the above problem, the present invention has adopted a configuration in which a sealing member, which separates a space in an apparatus body into the side of an ignition device and the side of a piston portion, seals a combustion product generated by the ignition device in the space on the side of the ignition device. With this configuration, it is possible to prevent the combustion product from being mixed into a substance to be administered. In addition, the sealing member is formed of a metal plate-like member, whereby the sealing member is suitably deformed by gunpowder combustion in the ignition device, and the shape of the sealing member after the deformation is easily maintained because the sealing member is made of metal. As a result, it is possible to transmit combustion energy of the gunpowder to the substance to be administered more reliably.

Specifically, the present invention is an administration apparatus that administers a substance to be administered to an administration target area, and the administration apparatus includes: an apparatus body having a through hole that is formed in an axial direction; a piston portion disposed so as to be slidable in the through hole; a syringe portion disposed on a side of a tip of the apparatus body, the syringe portion having an accommodation chamber that is capable of accommodating the substance to be administered, a plunger that pressurizes the substance to be administered in the accommodation chamber in response to a slide of the piston portion, and a nozzle portion that includes a channel in which the substance to be administered in the accommodation chamber pressurized by the plunger flows, and ejects the substance to be administered from an ejection outlet formed at a tip of the channel; an ignition device that is disposed on a side of a base end of the apparatus body, that combusts gunpowder, and that supplies ejection energy for ejecting the substance to be administered from the nozzle portion using gunpowder combustion in the ignition device; and a sealing member that is formed of a metal plate-like member, that separates a space in the apparatus body into a first space in which the ignition device is disposed and a second space in which the piston portion is disposed, and that seals a combustion product generated by the ignition device in the first space. A peripheral edge portion of the sealing member is fixed to an inner wall that defines the space in the apparatus body, and the sealing member is deformed by the gunpowder combustion in the ignition device such that a central portion of the sealing member is displaced to a side of the piston portion, to thereby press the piston portion against the plunger using the central portion.

In the administration apparatus according to the present invention, combustion energy generated by the gunpowder combustion in the ignition device is used as the ejection energy, and the central portion of the sealing member is deformed so as to be displaced to the side of the piston portion, whereby the piston portion is pressed against the plunger and slides in the through hole. Subsequently, pressure is applied to the substance to be administered accommodated in the accommodation chamber of the syringe portion via the plunger by the slide of the piston portion, and the substance to be administered is thereby ejected to the outside of the apparatus from the ejection outlet. Note that, in the case where the displacement of the central portion of the sealing member is caused by the combustion of the gunpowder, it is possible to appropriately adopt configurations such as a configuration in which the ejection energy is caused to directly act on the piston portion via the central portion of the sealing member, and a configuration in which the ejection energy is caused to propagate to another gas, liquid, or solid once and is then caused to indirectly act on the piston portion via the central portion.

As long as the substance to be administered can be ejected by using the ejection energy, the accommodation state of the substance to be administered in the administration apparatus may be any accommodation state, and the specific physical form of the substance to be administered such as fluid such as liquid or gel, powder, or a granular solid may be any physical form. The substance to be administered may contain an ingredient that is expected to be efficacious inside the administration target area. For example, in the case where the administration apparatus is an injector that injects the substance to be administered into a living body area, the substance to be administered contains an ingredient to be delivered to the administration target area of the living body, and the ingredient may be present in a state in which the ingredient is dissolved in the substance to be administered or in a state in which the ingredient is not dissolved therein but is simply mixed with the substance to be administered. Examples of the ingredient to be delivered include vaccine for strengthening an antibody, protein for beauty treatment, and cultured cells for hair regeneration. The substance to be administered is formed by causing fluid such as liquid or gel to contain the above ingredient such that the ingredient can be ejected. The injector may be a type of the injector that supplies the substance to be administered to the administration target area via a needle, or may also be a type of the injector that supplies the substance to be administered to the administration target area without the intervention of the needle.

In the administration apparatus according to the present invention, the ignition device that combusts the gunpowder may be the ignition device in which the gunpowder accommodated in the ignition device is ignited by execution of the ignition device and the combustion product of the gunpowder is generated, and may also be the ignition device in which a known gas generating agent (e.g., single-base smokeless gunpowder) is further combusted by the ignition of the gunpowder and the combustion products of the gunpowder and the gas generating agent are generated, and the specific configuration of the ignition device is not limited in the administration apparatus of the present invention.

When the gunpowder is combusted in the above ignition device, the combustion product is diffused in the space in the apparatus body and typically transmits the ejection energy to the piston portion via pressure or heat, and, as described above, the energy serves as a power source for ejection of the substance to be administered. The administration apparatus according to the present invention includes the sealing member, and hence the combustion product is sealed in the first space, and does not enter the second space. Consequently, it is possible to prevent unfavorable action of the combustion product on the substance to be administered. In order to obtain the sealing effect, the sealing member needs to have a certain level of resistance to the combustion of the gunpowder. On the other hand, it is not preferable that the transmission of the ejection energy to the piston portion be hindered by the presence of the sealing member. Accordingly, the sealing member needs to achieve both of suitable sealing of the combustion product and suitable transmission of the ejection energy to the piston portion.

To cope with this, the sealing member provided in the administration apparatus of the present invention is formed of the metal plate-like member. The sealing member is formed of the plate-like member, whereby, when the gunpowder is combusted in the ignition device, the generated combustion energy can be received by the surface of the plate-like member. As a result, the displacement amount of the central portion of the sealing member is increased, and it becomes easier to press the piston portion. Further, the sealing member is made of metal, whereby, in the case where the sealing member receives the combustion energy of the gunpowder and is deformed, the state after the deformation is maintained more easily than in the case where the sealing member is made of, e.g., an elastic resin such as rubber. That is, when the metal sealing member receives the combustion energy of the gunpowder, the metal sealing member is plastically deformed and does not return to its original state easily, and it becomes possible to substantially maintain the deformed state. This is extremely useful in the administration apparatus that transmits the combustion energy to the side of the piston portion via the sealing member and uses the combustion energy as the ejection energy for the substance to be administered. This is because the returning of the sealing member to the original state after the deformation means that the combustion energy to be transmitted to the side of the piston portion is lost. In view of the foregoing, in the administration apparatus according to the present invention, it becomes possible to achieve both of the sealing of the combustion product and the suitable transmission of the ejection energy to the piston portion.

The administration apparatus described above may be configured such that the sealing member is curved such that the central portion is positioned on a side of the ignition device with respect to the peripheral edge portion in a state before the gunpowder combustion in the ignition device, and the sealing member is curved such that the central portion is positioned on a side opposite to the side of the ignition device with respect to the peripheral edge portion by the gunpowder combustion in the ignition device. Thus, when the sealing member is deformed such that the position of the central portion of the sealing member with respect to the peripheral edge portion is displaced from the side of the ignition device, which is the side before the gunpowder combustion in the ignition device, to the side opposite to the side of the ignition device after the gunpowder combustion in the ignition device, it is possible to secure the large displacement amount of the sealing member in the direction of the slide of the piston portion at the time of the combustion. As a result, it becomes possible to efficiently transmit the ejection energy to the piston portion via the sealing member.

In addition, in the administration apparatus described above, with approach to the central portion of the sealing member from the ignition device along a central axis of the apparatus body, a cross-sectional area of the space in the apparatus body in a direction perpendicular to the central axis may be reduced. According to this configuration, it is possible to apply the combustion energy of the gunpowder in the ignition device intensively to the sealing member. As a result, it is possible to secure the large displacement amount of the central portion of the sealing member by the gunpowder combustion. In addition, in the case where the large displacement amount of the central portion is secured, as described above, the sealing member is made of metal, and hence the central portion is formed so as not to return to the original state easily, and the efficient transmission of the ejection energy to the piston portion is thereby implemented.

Further, in the administration apparatus described above, the ignition device may have a release portion that releases the combustion product generated by the gunpowder combustion and, in this case, the central portion of the sealing member is disposed so as to face the release portion. According to this configuration, it is possible to effectively apply the combustion energy of the gunpowder to the central portion of the sealing member, which contributes to the efficient transmission of the ejection energy to the piston portion.

Furthermore, in the administration apparatus described above, an end portion of the piston portion may be in contact with the central portion of the sealing member in the state before the gunpowder combustion in the ignition device. According to this configuration, it is possible to efficiently transmit the energy from the sealing member that receives the combustion energy of the gunpowder and is deformed to the piston portion.

### [Advantageous Effects of Invention]

According to the present invention, in the administration apparatus that administers the substance to be administered using the gunpowder combustion, it is possible to prevent the combustion product generated by the gunpowder combustion from acting on the substance to be administered, and suitably transmit energy for administration to the substance to be administered.

### [Brief Description of Drawings]

[Fig. 1]

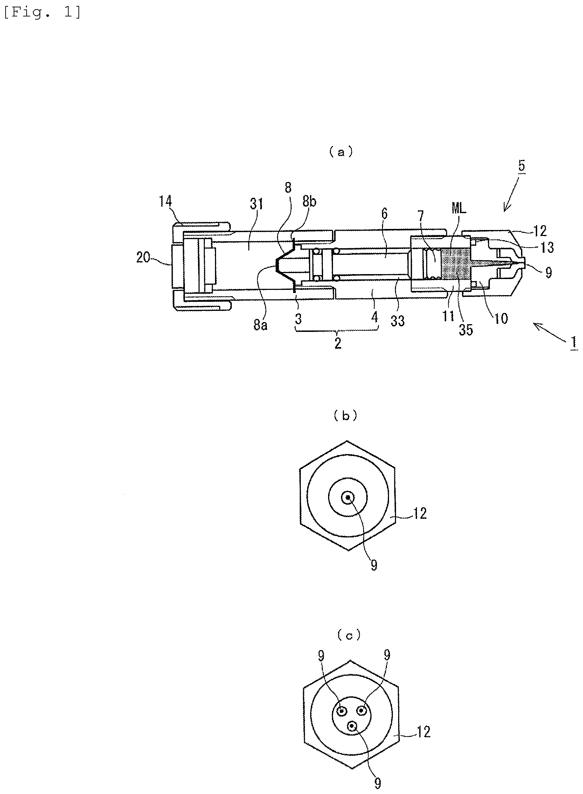

   Fig. 1 is a view showing the schematic configuration of an injector according to a first embodiment of the present invention.
[Fig. 2]

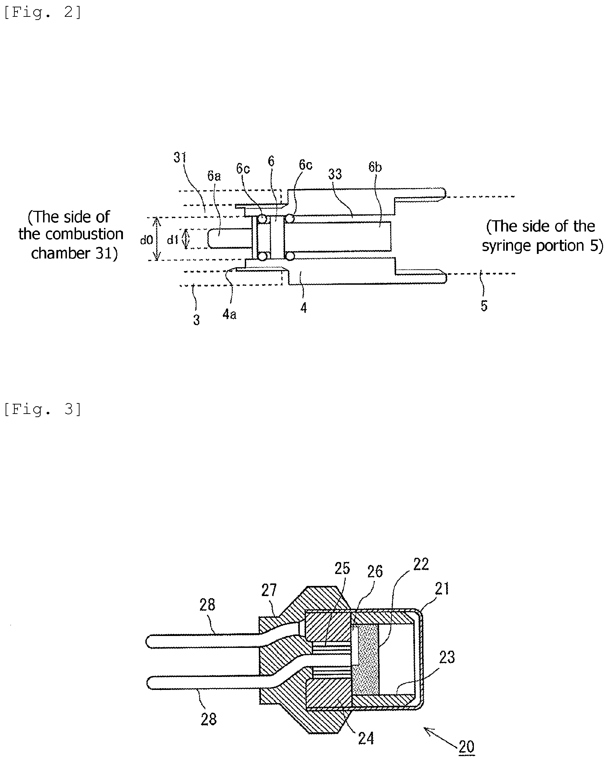

   Fig. 2 is a view showing the details of a piston of the injector shown in Fig. 1.
[Fig. 3]
   Fig. 3 is a view showing the schematic configuration of an initiator (ignition device) mounted to the injector shown in Fig. 1.
[Fig. 4]

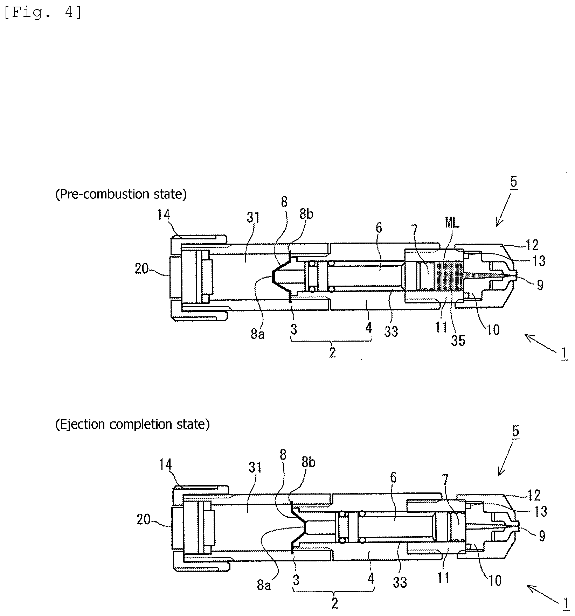

   Fig. 4 is a view in which, in the injector shown in Fig. 1, a state before gunpowder combustion in the initiator and a state after gunpowder combustion therein are compared with each other and shown.
[Fig. 5]

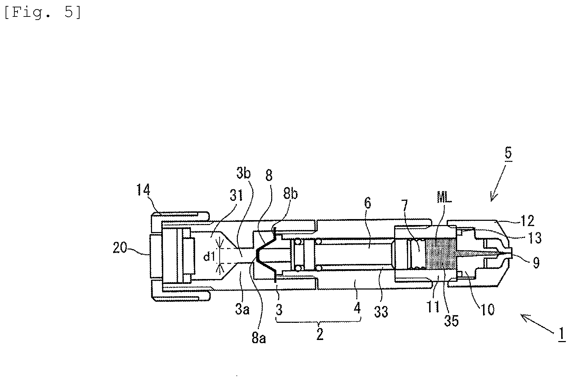

   Fig. 5 is a view showing the schematic configuration of the injector according to a second embodiment of the present invention.

### [Description of Embodiments]

Hereinbelow, description will be made by taking, as an example, a needleless injector 1 (hereinafter simply referred to as an "injector 1") with no injection needle that serves as an example of an administration apparatus according to the present invention with reference to the drawings. Note that the configurations of the following embodiments are shown by way of example only, and the present invention is not limited to the configurations of the embodiments.

### <First Embodiment>

Fig. 1(a) is a cross-sectional view of the injector 1, and Fig. 1(b) is a view when the injector 1 is viewed from the side of a nozzle portion 9 from which an injection solution is ejected. Note that, in the following description of the present application, a substance to be injected that is injected into an injection target area of an object by the injector 1 is collectively referred to as an "injection solution". That is, the substance to be injected is the substance that is administered to the injection target area, and corresponds to a substance to be administered of the present invention. However, this is not intended to limit the content and form of the injected substance. In the substance to be injected, an ingredient to be delivered to a skin structure may or may not be dissolved. In addition, as long as the substance to be injected can be ejected to the skin structure from the nozzle portion 9 by pressurization, the specific form of the substance to be injected may be any form, it is possible to use various forms such as liquid, gel, and gunpowder.

The injector 1 has an injector body 2 constituted by a first housing 3 and a second housing 4, and a syringe portion 5 is disposed on the side of the tip of the injector body 2 (the side of an end portion of the second housing 4 opposite to an end portion thereof connected to the first housing 3). The first housing 3 and the second housing 4 are fixed to each other with a screw and are thereby integrated with each other. Inside the first housing 3, a combustion chamber 31 that is an internal space extending in an axial direction of the first housing 3 is formed and, inside the second housing 4, a through hole 33 that is an internal space extending similarly in an axial direction of the second housing 4 is formed. Although the combustion chamber 31 and the through hole 33 are separated from each other by a sealing member 8, they are the internal spaces that are continuously disposed inside the injector body 2. The sealing member 8 is formed of a metal plate-like member. Note that the sealing member 8 also serves as a member that transmits energy of gunpowder combustion in an initiator 20 serving as an ignition device described later to a piston 6 as ejection energy, and the movement of the sealing member 8 at the time of the transmission will be described later.

The syringe portion 5 provided on the side of the tip of the injector body 2 has a syringe portion body 11 that has an accommodation chamber 35 that accommodates an injection solution ML inside the syringe portion body 11, the nozzle portion 9 in which a channel in which the injection solution flows is formed, and a nozzle body portion 10 in which the nozzle portion 9 is provided. The nozzle body portion 10 is mounted, via a gasket 13, to the syringe portion body 11 by a nozzle holder 12. The syringe portion body 11 is screwed with and mounted to the end portion of the second housing 4 of the injector body 2 and, in the mounting state, the through hole 33 in the second housing 4 and the accommodation chamber 35 in the syringe portion body 11 form continuous space. Note that, in the mounting state, the injection solution ML is accommodated in the accommodation chamber 35 fluid-tightly by a plunger 7, and the plunger 7 is exposed on the side of the through hole 33. The plunger 7 is disposed so as to be slidable in the accommodation chamber 35 and pressurizes the injection solution ML by sliding, and ejection of the injection solution from the nozzle portion 9 is thereby performed. The plunger 7 is formed of a rubber member, the surface of which is thinly coated with silicon oil so as to be able to smoothly slide in the accommodation chamber 35.

Next, the metal piston 6 is disposed in the through hole 33 in the second housing 4 of the injector body 2, and is held so as to be slidable in the through hole 33. Fig. 2 shows the details of the piston 6 such that the positional relationship between the piston 6 and the second housing 4 can be grasped. The piston 6 is formed into a substantially shaft-like shape that extends along an axial direction of the through hole 33, and has an end portion (hereinafter referred to as a "first end portion") 6a on the side of the combustion chamber 31 and an end portion on the side of the syringe portion 5, i.e., an end portion (hereinafter referred to as a "second end portion") 6b that comes into contact with the plunger 7 disposed in the syringe portion 5, and O-rings 6c are disposed around the piston 6 such that the piston 6 can slide smoothly in the through hole 33. In a state in which the injector body 2 is formed by mounting the first housing 3 (indicated by a dotted line in Fig. 2) and the second housing 4 to each other, and the gunpowder combustion is not yet performed in the initiator 20 (hereinafter referred to as a "pre-ignition state"), the first end portion 6a is substantially protruded to the side of the combustion chamber 31 from an end surface of a fitted portion 4a of the second housing 4 that is fitted in the combustion chamber 31 of the first housing 3. A diameter d1 of the first end portion 6a is smaller than a diameter d0 of the through hole 33. Consequently, when the piston 6 slides to the side of the syringe portion 5 in the through hole 33, a specific gap is formed between a side surface (a surface extending along an axial direction of the piston 6) of the first end portion 6a and an inner wall surface of the through hole 33.

In the pre-ignition state shown in Fig. 1, the sealing member 8 is fixed such that a peripheral edge portion 8b of the sealing member 8 is disposed on the end surface of the fitted portion 4a of the second housing 4 that is part of an inner wall of the injector body 2, and the peripheral edge portion 8b is buried in the first housing 3 in the vicinity of the fitted portion 4a. In addition, in the pre-ignition state, a central portion 8a of the sealing member 8 is in contact with an end surface of the first end portion 6a of the piston 6. As described above, in the pre-ignition state, the first end portion 6a is disposed so as to be protruded in the first housing 3, and hence, as a result, the sealing member 8 is fixed in the injector body 2 in a state in which the sealing member 8 is curved to the side of the initiator 20 in the pre-ignition state. The sealing member 8 is the plate-like member formed of a metal material and separates the space in the injector body 2 into a space including the combustion chamber 31 positioned on the side of the initiator 20 (corresponds to a first space according to the present invention) and a space including the through hole 33 positioned on the side of the piston 6 (corresponds to a second space according to the present invention), and a combustion product generated by the gunpowder combustion in the initiator 20 is thereby sealed in the combustion chamber 31. Note that the movement of the sealing member 8 by the gunpowder combustion in the initiator 20 will be described later.

An example of the initiator 20 will be described based on Fig. 3. The initiator 20 is an electrical ignition device, and a space for disposing gunpowder 22 is defined in a cup 21 by the cup 21, the surface of which is covered with an insulating cover. A metal header 24 is disposed in the space, and a tubular charge holder 23 is provided on an upper surface of the metal header 24. The gunpowder 22 is held by the charge holder 23. A bridge wire 26 that electrically connects one of conductive pins 28 and the metal header 24 is disposed at a bottom portion of the gunpowder 22. Note that the two conductive pins 28 are fixed to the metal header 24 via an insulator 25 so as to be insulated from each other when voltage is not applied. Further, an open port of the cup 21 from which the two conductive pins 28 supported by the insulator 25 extend is protected by a resin collar 27 in a state in which insulation characteristics between the conductive pins 28 are properly maintained.

In the thus configured initiator 20, when a voltage is applied between the two conductive pins 28 by an external power source, a current flows to the bridge wire 26, and the gunpowder 22 is thereby combusted. At this point, the combustion product generated by the combustion of the gunpowder 22 is jetted from an opening portion of the charge holder 23. The cross section of an initiator cap 14 is formed into a flange-like shape such that the initiator cap 14 is caught on an outer surface of the initiator 20, and the initiator cap 14 is fixed to the first housing 3 by using a screw. With this, the initiator 20 is fixed to the first housing 3 by using the initiator cap 14, and it is possible to prevent the initiator 20 itself from being disconnected from the injector body 2 by pressure generated at the time of the ignition in the initiator 20.

An example of the gunpowder 22 used in the injector 1 preferably includes a gunpowder containing zirconium and potassium perchlorate (ZPP), a gunpowder containing titanium hydride and potassium perchlorate (THPP), a gunpowder containing titanium and potassium perchlorate (TiPP), a gunpowder containing aluminum and potassium perchlorate (APP), a gunpowder containing aluminum and bismuth oxide (ABO), a gunpowder containing aluminum and molybdenum oxide (AMO), a gunpowder containing aluminum and copper oxide (ACO), a gunpowder containing aluminum and iron oxide (AFO), or a gunpowder obtained by combining a plurality of the gunpowders. Each gunpowder exhibits a characteristic that the gunpowder generates high-temperature high-pressure plasma during the combustion immediately after the ignition but, when the temperature is reduced to normal temperature and the combustion product condenses, generated pressure is sharply reduced because no gas component is contained. Note that another gunpowder other than the gunpowders described above may be used.

Nothing is disposed in the combustion chamber 31 shown in Fig. 1, but a gas generating agent that is combusted by the combustion product generated by the combustion of the gunpowder 22 and generates gas may be disposed in the combustion chamber 31. In the case where the gas generating agent is disposed in the combustion chamber 31, an example of the gas generating agent includes single-base smokeless gunpowder containing 98 mass% of nitrocellulose, 0.8 mass% of diphenylamine, and 1.2 mass% of potassium sulfate. In addition, it is also possible to use various gas generating agents that are used in a gas generator for an air bag and a gas generator for a seat belt pretensioner. In the combined use of the gas generating agent and the gunpowder, unlike the case where only the gunpowder 22 is used, specific gas generated during the combustion contains the gas component even at normal temperature, and hence the reduction rate of the generated pressure is low. Further, combustion completion time at the time of the combustion of the gas generating agent is significantly longer than that of the above gunpowder 22, but it is possible to change the combustion completion time of the gas generating agent by adjusting the dimensions, the size, the shape, and particularly the surface shape of the gas generating agent when the gas generating agent is disposed in the combustion chamber 31. Thus, by adjusting the amount, the shape, and the disposition of the gas generating agent, it is possible to appropriately adjust the generated pressure in the combustion chamber 31.

Note that a plurality of the nozzle portions 9 may be formed in the nozzle body portion 10, or one nozzle portion 9 may be formed in the nozzle body portion 10. In the case where a plurality of the nozzle portions are formed, the channels corresponding to the individual nozzle portions are formed such that the released injection solution ML is sent to the individual nozzles as uniformly as possible. Further, in the case where a plurality of the nozzle portions 9 are formed, as shown in Fig. 1(c), it is preferable that the individual nozzle portions be disposed so as to be exposed at regular intervals around the central axis of the injector 1, and be fixed by the nozzle holder 12. The diameter of the channel of the nozzle portion 9 is configured to be smaller than the inner diameter of the through hole 33. With this, it is possible to suitably increase the ejection pressure of the injection solution at the time of the ejection.

The ejection state of the injection solution in the injector 1 will be described based on Fig. 4 together with the movement of each of the sealing member 8 and the piston 6 when the gunpowder 22 in the initiator 20 is combusted. The upper part of Fig. 4 shows the configuration of the injector 1 in the pre-ignition state, while the lower part of Fig. 4 shows the configuration of the injector 1 in a state in which the ejection of the injection solution is completed (hereinafter referred to as an "ejection completion state").

In a pre-combustion state, the peripheral edge portion 8b is disposed on the end surface of the fitted portion 4a and the sealing member 8 is fixed on the side of the first housing 3 and, at this point, the sealing member 8 is curved such that the central portion 8a of the sealing member 8 is positioned on the side of the initiator 20 with respect to the peripheral edge portion 8b, and the central portion 8a of the sealing member 8 is in contact with the end surface of the first end portion 6a of the piston 6. In addition, the central portion 8a of the sealing member 8 is positioned so as to face the cup 21 of the initiator 20. With this disposition, the sealing member 8 receives pressure by the combustion product released from the initiator 20 using its plate-like surface. In particular, the central portion 8a of the sealing member 8 is disposed at the position where the central portion 8a receives the pressure directly, and hence, as will be described later, it becomes easier for the central portion 8a to press the piston 6.

When the gunpowder 22 is combusted in the initiator 20, the combustion product is diffused in the combustion chamber 31, and the pressure in the combustion chamber 31 is increased. With this, the pressure is also applied to the sealing member 8. At this point, the central portion 8a is positioned in the central part of the plate-like sealing member 8, and hence the central portion 8a is the portion that is most likely to be deformed in the sealing member 8 of which the peripheral edge portion 8b is fixed. Accordingly, when the pressure is applied to the sealing member 8 by the gunpowder combustion, the sealing member 8 is deformed such that the central portion 8a presses the first end portion 6a of the piston 6 to the side of the tip of the injector 1. In other words, the sealing member 8, which is curved such that the central portion 8a is positioned on the side of the initiator 20 with respect to the peripheral edge portion 8b, is deformed so as to be curved such that the central portion 8a is positioned on the side opposite to the side of the initiator 20 by the gunpowder combustion. By causing the central portion 8a to move in response to the movement of the sealing member 8, it is possible to secure the large displacement amount of the central portion 8a of the sealing member 8 by the gunpowder combustion, and efficiently press the injection solution ML. Further, in the pre-combustion state, the central portion 8a is in contact with the first end portion 6a, and hence it is possible to effectively convert the displacement of the central portion 8a of the sealing member 8 by the gunpowder combustion to the slide of the piston 6.

In addition, the sealing member 8 is formed of the metal material, and hence, when the pressure is applied sharply to the sealing member 8 by the gunpowder combustion in the initiator 20, the sealing member 8 is substantially plastically deformed when the central portion 8a is displaced from the side of the initiator 20 to the side opposite to the side of the initiator 20 with respect to the peripheral edge portion 8b, as described above (see the ejection completion state in Fig. 4). That is, when the sharp pressure change is caused by the gunpowder combustion, the pressure that exceeds the yield point of the metal material forming the sealing member 8 is applied to the sealing member 8, and the sealing member 8 is plastically deformed. This means that the central portion 8a of the sealing member 8, which has been displaced to the side opposite to the side of the initiator 20 with respect to the peripheral edge portion 8b, cannot easily return to the side of the initiator 20. Consequently, when the sealing member 8 presses the piston 6 using the gunpowder combustion, the sealing member 8 can be deformed while adequately resisting reaction from the piston 6, and hence it becomes possible to effectively pressurize the injection solution ML via the piston 6 and the plunger 7.

In the injector 1 configured to allow the ejection of the injection solution ML described above, the piston 6 is pressed by the sealing member 8 and the entire piston 6 is thereby caused to slide, whereby the ejection energy is transmitted directly to the plunger 7. In particular, the sealing member 8 is made of metal and is formed into the plate-like shape, and hence the effective pressurization of the injection solution ML by the central portion 8a is allowed. In addition, in the injector 1, the sealing member 8 is disposed so as to separate the combustion chamber 31 and the through hole 33 that are the internal spaces of the injector body 2 from each other, and seal the combustion product generated by the initiator 20 in the combustion chamber 31. With this configuration, the combustion product does not enter the through hole 33, whereby it is possible to prevent unfavorable action of the combustion product on the injection solution ML.

### <Second Embodiment>

Fig. 5 shows a second embodiment of the present invention. Fig. 5 shows the schematic configuration of the injector 1 according to the present embodiment in the pre-combustion state. Note that, in the present embodiment, configurations substantially identical to those in the first embodiment described above are designated by the same reference numerals, and the detailed description thereof will be omitted.

In the injector 1 of the present embodiment, a narrowed portion 3a is formed inside the first housing 3 such that the inner diameter of the combustion chamber 31 formed inside the first housing 3 is reduced with approach to the central portion 8a of the sealing member 8 from the side of the initiator 20 along the central axis of the injector 1. With the narrowed portion 3a, the inner diameter of the combustion chamber 31 at a position where the inner diameter is smallest becomes substantially equal to d1 that is the outer diameter of the first end portion 6a of the piston 6, and a passage 3b having a diameter of about d1 is formed in the combustion chamber 31 at a position facing the first end portion 6a. In the case where the combustion chamber 31 is formed in this manner, when the gunpowder is combusted in the initiator 20, it becomes easier for the pressure by the combustion product to act intensively on the central portion 8a of the sealing member 8. As a result, it becomes possible to efficiently bring the sealing member 8 in the pre-combustion state into the ejection completion state shown in Fig. 4, and the amount of gunpowder used in the initiator 20 can be thereby reduced.

Note that, in order to allow the combustion product concentrated as described above to act on the central portion 8a of the sealing member 8 that is in contact with the first end portion 6a of the piston 6, it is preferable that a clearance between an end portion of the passage 3b on the side of the piston 6 and the central portion 8a of the sealing member 8 with which the first end portion 6a is in contact be as small as possible. Also by forming the narrowed portion 3a such that the inner diameter of the passage 3b is smaller than d1, it becomes possible to cause the combustion product to act on the central portion 8a of the sealing member 8 more intensively.

### <Other Embodiments>

According to the injector 1 of the present invention, in addition to the above-described case where the injection solution is injected into the skin structure, for example, in the field of regenerative medicine for humans, it becomes possible to inoculate cultured cells or stem cells into cells and scaffold tissue (scaffold) serving as injection targets. For example, as described in Japanese Patent Application Publication No. 2008-206477, it is possible to inject, by using the injector 1, cells that can be appropriately determined by those skilled in the art according to a portion subjected to transplantation and the purpose of cell regeneration such as, e.g., an endothelial cell, an endothelial precursor cell, a myeloid cell, a preosteoblast cell, a chondrocyte cell, a fibroblast cell, a skin cell, a muscle cell, a liver cell, a kidney cell, an intestinal tract cell, and a stem cell, as well as every cell considered in the field of regenerative medicine. More specifically, by accommodating a solution (cell suspension) containing the cell to be inoculated in the accommodation chamber 35 and pressurizing the solution, a specific cell is injected and transplanted into the portion subjected to transplantation.

Further, the injector 1 according to the present invention can be used for delivery of DNA or the like to cells and scaffold tissue (scaffold) described in Japanese Translation of PCT Application No. 2007-525192. In this case, the use of the injector 1 according to the present invention is more preferable than the use of a needle in the delivery because the injector 1 can reduce influences on cells and scaffold tissue (scaffold).

Further, the injector 1 according to the present invention is suitably used in the case where various genes, cancer suppressing cells, or lipid envelopes are directly delivered to target tissue and in the case where an antigen gene is administered in order to enhance immunity against a pathogen. In addition, the injector 1 can also be used in the field of medical treatment of various diseases (field described in Japanese Translation of PCT Application No. 2008-508881 or Japanese Translation of PCT Application No. 2010-503616) and the field of immunological medical treatment (field described in Japanese Translation of PCT Application No. 2005-523679), and the field in which the injector 1 can be used is not intentionally limited.

Another example of the administration apparatus to which the present invention can be applied includes a catheter device. The catheter device has a catheter portion that can enter a living body, and is a device that ejects a desired chemical solution or the like to the living body from its tip portion. The invention of the present application can be applied to the configuration of the chemical solution ejection from the tip portion of the catheter portion. That is, in a state in which the catheter portion is inside the living body, by applying the present invention to the configuration thereof for the ejection of the chemical solution from the tip portion, it is possible to efficiently transmit energy by the gunpowder combustion in the initiator 20 to the chemical solution, and prevent the combustion product from acting on the chemical solution.

### [Reference Signs List]

- 1: Injector
- 2: Injector body
- 3b: Passage
- 5: Syringe portion
- 6: Piston
- 7: Plunger
- 8: Sealing member
- 8a: Central portion
- 8b: Peripheral edge portion
- 9: Nozzle portion
- 10: Nozzle holder
- 11: Syringe portion body
- 20: Initiator
- 22: Gunpowder
- 31: Combustion chamber
- 33: Through hole
- 35: Accommodation chamber

## Claims

1. An administration apparatus (1) that administers a substance to be administered (ML) to an administration target area, and the administration apparatus comprising:
an apparatus body (2) having a through hole (33) that is formed in an axial direction;
a piston portion (6) disposed so as to be slidable in the through hole;
a syringe portion (5) disposed on a side of a tip of the apparatus body, the syringe portion having an accommodation chamber (35) that is capable of accommodating the substance to be administered, a plunger (7) that pressurizes the substance to be administered in the accommodation chamber in response to a slide of the piston portion, and a nozzle portion (9) that includes a channel in which the substance to be administered in the accommodation chamber pressurized by the plunger flows, and ejects the substance to be administered from an ejection outlet formed at a tip of the channel; an ignition device (20) that is disposed on a side of a base end of the apparatus body, that combusts gunpowder, and that supplies ejection energy for ejecting the substance to be administered from the nozzle portion using gunpowder combustion in the ignition device; and
a sealing member (8) that is formed of a metal plate-like member, that separates a space in the apparatus body into a first space (31) in which the ignition device is disposed and a second space in which the piston portion is disposed, and that seals a combustion product generated by the ignition device in the first space, wherein a peripheral edge portion (8b) of the sealing member is fixed to an inner wall that defines the space in the apparatus body, and the sealing member is deformed by the gunpowder combustion in the ignition device such that a central portion (8a) of the sealing member is displaced to a side of the piston portion, to thereby press the piston portion against the plunger using the central portion.

2. The administration apparatus according to claim 1, wherein the sealing member is curved such that the central portion is positioned on a side of the ignition device with respect to the peripheral edge portion in a state before the gunpowder combustion in the ignition device, and
the sealing member is curved such that the central portion is positioned on a side opposite to the side of the ignition device with respect to the peripheral edge portion by the gunpowder combustion in the ignition device.

3. The administration apparatus according to claim 1 or 2, wherein
with approach to the central portion of the sealing member from the ignition device along a central axis of the apparatus body, a cross-sectional area of the space in the apparatus body in a direction perpendicular to the central axis is reduced.

4. The administration apparatus according to any one of claims 1 to 3, wherein
the ignition device has a release portion that releases the combustion product generated by the gunpowder combustion and,
the central portion of the sealing member is disposed so as to face the release portion.

5. The administration apparatus according to any one of claims 1 to 4, wherein
an end portion (6a) of the piston portion is in contact with the central portion of the sealing member in the state before the gunpowder combustion in the ignition device.

## Patentansprüche

1. Verabreichungsvorrichtung (1), die eine zu verabreichende Substanz (ML) in einen Verabreichungszielbereich verabreicht, wobei die Verabreichungsvorrichtung folgendes umfasst:
einen Vorrichtungskörper (2) mit einem Durchgangsloch (33), das in einer axialen Richtung ausgebildet ist;
einen Kolbenabschnitt (6), der so angeordnet ist, dass er in dem Durchgangsloch verschiebbar ist;
einen Spritzenabschnitt (5), der auf einer Seite einer Spitze des Vorrichtungskörpers angeordnet ist, wobei der Spritzenabschnitt eine Aufnahmekammer (35), die die zu verabreichende Substanz aufnehmen kann, einen Plunger (7), der die zu verabreichende Substanz in der Aufnahmekammer als Reaktion auf eine Verschiebung des Kolbenabschnitts unter Druck setzt, und einen Düsenabschnitt (9) aufweist, der einen Kanal aufweist, in dem die zu verabreichende Substanz in der Aufnahmekammer, welche durch den Kolben unter Druck gesetzt wird, fließt und der die zu verabreichende Substanz aus einem an einer Spitze des Kanals ausgebildeten Ausstoßauslass ausstößt;
eine Zündvorrichtung (20), die an einer Seite eines Basisendes des Vorrichtungskörpers angeordnet ist, die Schießpulver verbrennt und die unter Verwendung von Schießpulververbrennung in der Zündvorrichtung Ausstoßenergie zum Ausstoßen der zu verabreichenden Substanz aus dem Düsenabschnitt liefert; und
ein Dichtungselement (8), das aus einem metallplattenartigen Element gebildet ist, das einen Raum in dem Vorrichtungskörper in einen ersten Raum (31), in dem die Zündvorrichtung angeordnet ist, und einen zweiten Raum, in dem der Kolbenabschnitt angeordnet ist, unterteilt, und das ein von der Zündvorrichtung in dem ersten Raum erzeugtes Verbrennungsprodukt abdichtet, wobei ein Umfangsrandabschnitt (8b) des Dichtungselements an einer Innenwand befestigt ist, die den Raum in dem Vorrichtungskörper definiert, und wobei das Dichtungselement durch die Schießpulververbrennung in der Zündvorrichtung so verformt wird, dass ein zentraler Abschnitt (8a) des Dichtungselements zu einer Seite des Kolbenabschnitts verschoben wird, um dadurch den Kolbenabschnitt unter Verwendung des zentralen Abschnitts gegen den Plunger zu drücken.

2. Verabreichungsvorrichtung nach Anspruch 1, wobei
das Dichtungselement so gekrümmt ist, dass in einem Zustand vor der Schießpulververbrennung der zentrale Abschnitt in Bezug auf den Umfangsrandabschnitt auf einer Seite der Zündvorrichtung positioniert ist, und
das Dichtungselement so gekrümmt ist, dass der zentrale Abschnitt in Bezug auf den Umfangsrandabschnitt durch die Schießpulververbrennung in der Zündvorrichtung auf einer Seite gegenüber der Seite der Zündvorrichtung positioniert wird.

3. Verabreichungsvorrichtung nach Anspruch 1 oder 2, wobei
bei Annäherung an den zentralen Abschnitt des Dichtungselements von der Zündvorrichtung aus entlang einer zentralen Achse des Vorrichtungskörpers eine Querschnittsfläche des Raums in dem Vorrichtungskörper in einer Richtung senkrecht zu der zentralen Achse verringert wird.

4. Verabreichungsvorrichtung nach einem der Ansprüche 1 bis 3, wobei
die Zündvorrichtung einen Freigabeabschnitt aufweist, der das durch die Schießpulververbrennung erzeugte Verbrennungsprodukt freigibt, und
der zentrale Abschnitt des Dichtungselements so angeordnet ist, dass er dem Freigabeabschnitt zugewandt ist.

5. Verabreichungsvorrichtung nach einem der Ansprüche 1 bis 4, wobei
ein Endabschnitt (6a) des Kolbenabschnitts im Zustand vor der Schießpulververbrennung in der Zündvorrichtung mit dem zentralen Abschnitt des Dichtungselements in Kontakt ist.

## Revendications

1. Appareil d'administration (1) qui administre une substance à administrer (ML) dans une zone cible d'administration, l'appareil d'administration comprenant :
- un corps d'appareil (2) ayant un orifice traversant (33) formé dans la direction axiale,
- une partie-piston (6) disposée de façon à pouvoir coulisser dans l'orifice,
- une partie-seringue (5) placée sur le côté d'une extrémité du corps de l'appareil, la partie-seringue ayant une chambre de réception (35) recevant la substance à administrer, un plongeur (7) qui met en pression la substance à administrer dans la chambre de réception, par le coulissement de la partie-piston ainsi qu'une partie-buse (9) ayant un canal dans lequel la substance à administrer est comprimée dans la chambre de réception par le plongeur et qui éjecte la substance à administrer par la sortie d'éjection formée à l'extrémité du canal,
- un dispositif d'allumage (20) placé sur un côté de l'extrémité de base du corps de l'appareil et qui contient de la poudre explosive fournissant l'énergie d'éjection pour éjecter la substance à administrer par la partie-buse en utilisant la combustion de la poudre dans le dispositif d'allumage, et
- un élément d'étanchéité (8) formé comme un élément analogue à une plaque métallique séparant le volume du corps de l'appareil en un premier espace (31) dans lequel se trouve le dispositif d'allumage et un second espace dans lequel se trouve la partie piston et qui assure l'étanchéité du produit de combustion généré par le dispositif d'allumage dans le premier espace,
- une partie de bord périphérique (8b) de l'élément d'étanchéité étant fixée à la paroi intérieure délimitant l'espace dans le corps de l'appareil, l'élément d'étanchéité étant déformé par la combustion de la poudre dans le dispositif d'allumage de façon que la partie centrale (8a) de l'élément d'étanchéité soit déplacée vers un côté de la partie-piston pour presser la partie-piston contre le plongeur par la partie centrale.

2. Appareil d'administration selon la revendication 1,
dans lequel
- l'élément d'étanchéité est courbé pour que la partie centrale soit sur un côté du dispositif d'allumage par rapport à la partie de bord périphérique dans l'état précédent la combustion de la poudre dans le dispositif d'allumage, et
- l'élément d'étanchéité est courbé pour que la partie centrale soit mise sur le côté opposé au côté du dispositif d'allumage par rapport à la partie de bord périphérique par la combustion de la poudre dans le dispositif d'allumage.

3. Appareil d'administration selon la revendication 1 ou 2,
dans lequel
la surface de la section de l'espace dans le corps de l'appareil diminue dans une direction perpendiculaire à l'axe central en s'approchant de la partie centrale de l'élément d'étanchéité à partir du dispositif d'allumage selon l'axe central du corps de l'appareil.

4. Appareil d'administration selon l'une quelconque des revendication 1 à 3,
dans lequel
- le dispositif d'allumage a une partie d'évacuation qui libère le produit de combustion généré par la combustion de la poudre, et
- la partie centrale de l'élément d'étanchéité est face à la partie d'évacuation.

5. Appareil d'administration selon l'une quelconque des revendication 1 à 4,
dans lequel
la partie d'extrémité (6a) de la partie-piston est en contact avec la partie centrale de l'élément d'étanchéité dans l'état qui précède la combustion de la poudre dans le dispositif d'allumage.
